# EUROPEAN PATENT APPLICATION

(11) **EP 2 940 133 A1**
(43) Date of publication of application: **04.11.2015**
(21) Application number: 13868570.6
(22) Date of filing: 28.11.2013
(51) Int. Cl.: C12N 15/09, A61K 39/00, A61K 48/00, A61P 31/20, A61P 35/00, C07K 14/02, C07K 14/025, C07K 19/00, C12N 1/15, C12N 1/19, C12N 1/21, C12N 5/10, A61K 39/12, A61K 39/29

(54) **VACCINE FOR HPV INFECTION AND/OR HEPATITIS B CONTAINING HPV/HBS CHIMERIC PROTEIN AS ACTIVE INGREDIENT**

(30) Priority: 25.12.2012 JP 2012280850
(71) Applicant: The Chemo-Sero-Therapeutic Research Institute, Kumamoto-shi Kumamoto 860-8568 (JP); Japan as represented by Director General of Agency of National Institute of Infectious Deseases, Shijuku-ku Tokyo 162-8640 (JP)
(72) Inventor: YONEMURA, Hiroshi, Kikuchi-shi Kumamoto 869-1298 (JP); SAKAGUCHI, Masashi, Kikuchi-shi Kumamoto 869-1298 (JP); IMAMURA, Takashi, Kikuchi-shi Kumamoto 869-1298 (JP); MORI, Seiichiro, Tokyo 162-8640 (JP); KANDA, Tadahito, Tokyo 162-8640 (JP)
(74) Representative: Vossius & Partner Patentanwälte Rechtsanwälte mbB
(86) International application number: PCT/JP2013/082094
(87) International publication number: WO 2014/103608

(57) **Abstract**

A chimeric protein of HPV-L2 peptide and HBs protein wherein the HPV-L2 peptide is (1) a peptide consisting of the core sequence region of 20 amino acid residues, (2) a peptide inside the core sequence region comprising 6 amino acid residues Gly-Gly-Leu-Gly-Ile-Gly or (3) a peptide consisting of 70 or less amino acid residues obtained by adding an amino acid sequence derived from HPV L2 protein at the N-terminal and/or the C-terminal of the core sequence region; and a vaccine for HPV infection and/or hepatitis B comprising the chimeric protein as an active ingredient. A vaccine comprising the chimeric protein of the present invention is the one that has an increased expression level in a host, an enhanced immune ability (early antibody induction) and a broader spectrum effective for a number of HPV types.

## Description

### TECHNICAL FIELD

The present invention relates to a vaccine comprising as an active ingredient a chimeric protein of a peptide derived from human papilloma virus (Human papillomavirus: HPV) and a human hepatitis B virus surface protein (HBs protein).

### BACKGROUND ART

Human papilloma virus (Human papillomavirus: HPV) is a small circular double-stranded DNA virus belonging to the papilloma virus family. HPV has an icosahedral structure with a diameter of 50-55 nm wherein the virus genome is covered in an envelope protein (Capsid protein) but does not have a coat covering the capsid. A genome size, though varies depending on types, is approximately 8,000 bases where early proteins (E1, E2, E4, E5, E6 and E7) and late proteins (L1 and L2) are encoded. Capsid of HPV particles is formed with L1 and L2 in which L2 is supplementary in capsid formation.

A host range of HPV is strict and HPV does not infect non-human animals. HPV, being latent in the skin and mucous membranes after contact infection, infects through persistent infection and causes cervical cancer (squamous cell carcinoma, adenocarcinoma) and their prodromal lesions [cervical intraepithelial neoplasia (CIN) 2 and 3], condylomata acuminata, and the like. In Japan, 19,000 people per year have developed cervical cancer, among which 5,600 people have died (cf. Non-Patent reference 5). According to the estimation by WHO, 13% of malignant tumors of women in all over the world (530,000 people) is associated with HPV infection and 300 million people is a carrier of HPV (cf. Non-Patent reference 4).

With respect to the study in viral growth, HPV genome replicates during division of HPV-infected cells and is distributed to daughter cells. When the latent infection cells begin differentiation of epidermis formation, viral growth occurs at the end of the differentiation. However, HPV is rarely separated as a viral particle and only the genomic DNA has been cloned. Based on the homology of nucleotide sequences of the capsid protein (L1) gene, HPV has ever been classified into 100 or more genotypes. HPV may infect at different sites and cause different diseases depending on its genotype. HPV is broadly divided into those detected in skin lesions (epithelial type) and those detected in mucosal lesions (mucosal type). Mucosal type HPV is further classified into a high-risk type detected in a variety of cancers and a low-risk type causative of benign condylomata acuminata. A high-risk type HPV includes types 16, 18, 26, 31, 33, 35, 39, 45, 51, 52, 53, 56, 58, 59, 66, 68, 73 and 82, detected in cervical cancer, perianal cancer and penile cancer whereas a low-risk type HPV includes types 6 and 11 (cf. Non-patent reference 1, Non-patent reference 2 and Non-patent reference 3).

With regard to a vaccine for prevention of HPV infection, "product name Gardasil (registered trademark)" developed by the US Merck & Co., Inc., a vaccine of a mixture of virus-like particle (VLP) of HPV types 6, 11, 16 and 18, has been approved by the Food and Drug Administration (FDA) in June 2006 and "product name Cervarix" developed by the UK GlaxoSmithKline, a vaccine of a mixture of VLP of HPV types 16 and 18, has been approved by the Therapeutic Goods Administration (TGA) of Australia in May 2007. In Japan, Cervarix has been approved in October 2009 and Gardasil in July 2011.

These vaccines, to be called a first generation vaccine, are prepared by expressing a gene encoding L1 protein in yeast or insect cells to produce the VLP, to which adjuvant is added (cf. Non-patent reference 6). Both vaccines have been approved for cancers caused by a particular genotype and for protective effects against only a few types, i.e. types 6, 11, 16 and 18 for Gardasil and types 16 and 18 for Cervarix (Non-patent reference 12). Thus, in order to block HPV infection and suppress more effectively the development of cancer subsequently triggered, the development of a vaccine effective for any types of HPV infection is desired (cf. Non-patent reference 5 and Non-patent reference 6).

As such an attempt, there is a report by Kanda et al. They showed that a neutralizing epitope common among the high-risk HPVs is found in the amino acid sequence of L2 of HPV type 16 at positions 64-81 and 108-120 and that VLP of aggregate of chimeric proteins obtained by inserting a peptide derived from L2 of HPV type 16 into L1 of HPV type 16 blocked infection of HPV types 16, 18, 31, 33, 35, 52 and 6 (cf. Patent reference 1 and Patent reference 2). In addition, they also reported the same results for VLP of chimeric proteins obtained by inserting L2 peptides at positions 18-38, 56-75 and 96-115 into L1 (cf. Non-patent reference 13). On the other hand, they also reported that the length of the inserted peptide may affect the presentation of the antigenic epitope portion (Patent reference 1).

Other than L1 of HPV, research on particle formation and utility as a vaccine of chimeric proteins obtained by adding or inserting a foreign peptide into a protein having a variety of particle-forming ability has been reported. For example, it has been confirmed that mice were immunized with a chimeric protein wherein the surface proteins (HBs protein) of hepatitis B virus (HBV) is added at its N-terminal or the C-terminal a peptide consisting of a part of L2 of HPV type 16 (50-220 amino acid region and 100-220 amino acid region from the N-terminal) to result in an increase in antibodies against L2 and HBs in ELISA (cf. Patent reference 7). There are other reports on VLP formation of a chimeric protein obtained by inserting a foreign gene into the hydrophilic region of HBs protein or the exposed portion of the outer loop of HBsAg-S (cf. Patent reference 3 and Patent reference 4) and of a chimeric protein wherein a foreign peptide is bound to the N-terminal of HBs protein (cf. Patent reference 5 and Patent reference 6).

HBs proteins have been proved to be highly effective as a vaccine and highly safe as they are used as an active ingredient of a recombinant precipitated hepatitis B vaccine derived from yeast (cf. Non-patent reference 7). However, VLP comprising a chimeric protein of a foreign peptide and the HBs protein, due to difference in the type and length of the foreign peptide to be inserted, which may affect the stability of VLP, the efficiency of expression from the cell and the structural change in what is expressed (cf. Patent reference 4), is not necessarily ensured for its usefulness as a vaccine.
Patent reference 1: WO2005/097987
Patent reference 2: JP Patent Publication No. 2007-45746
Patent reference 3: JP-A-8-198897
Patent reference 4: JP Patent Publication No. 2004-504067
Patent reference 5: JP Patent Publication No. 2011-115042
Patent reference 6: JP Patent Publication No. 2007-209343
Patent reference 7: WO2010/001409
Non-patent reference 1: Virus Vol. 56, No. 2, pp219-230, 2006
Non-patent reference 2: Igaku-No-Ayumi Vol.224, No9: 669-680, 2008
Non-patent reference 3: Munoz, N. et al.: New Engl J Med, 348, 518-527, 2003
Non-patent reference 4: Monitoring of Cancer Incidence in Japan MCIJ2007, March 2012
Non-patent reference 5: virus Vol. 58, No. 2, pp155-164, 2008
Non-patent reference 6: Sanka-To-Fujinka 73(2): 217-225, 2006
Non-patent reference 7: National Institute of Infectious Diseases, fact sheet on the hepatitis B vaccine (July 7, 2010 ed.)
Non-patent reference 8: Kawana. K, et al.: Common neutralization epitope in minor capsid protein L2 of human papillomavirus types 16 and 6, J. Virology., 73, 6188-6190, 1999 (106-121 of L2)
Non-patent reference 9: Ivonne Rubio et al.: Potent anti-HPV immune responses induced by tandem repeats of the HPV16 L2 (20-38) peptide displayed on bacterial thioredoxin Vaccine, 27, 1949-1956, 2009
Non-patent reference 10: Fujiyama, A. et al.: Nuc Acid Res., 11 (13), 4601-4610, 1983
Non-patent reference 11: Christophe, M. et al.: Emer. Inf. Dis., 14 (11), 1777-1780, 2008
Non-patent reference 12: Nippon Jibiinkoka Gakkai Kaiho Vol. 115 No. 2, 73-84, 2012
Non-patent reference 13: Kondo, K et al.: Journal of Medical Virology 80: 841-846 (2008)

### DISCLOSURE OF THE INVENTION

### (Technical Problem to be Solved by the Invention)

An object of the present invention is to provide a vaccine for preventing human papilloma virus infection and/or hepatitis B comprising as an active ingredient a chimeric protein obtained by adding a peptide consisting of a 20-amino acid sequence of a specific region of a protein called L2 (hereinafter, also referred to as "L2 protein"), one of the capsid components of HPV, to the N-terminal or the C-terminal of the HBs protein or by inserting the peptide inside the HBs protein.

### (Means for Solving the Problems)

The present inventors have found, as a result of extensive studies to achieve the above object, that a chimeric protein obtained by adding a peptide consisting of the amino acids at positions 56-75 of the L2 protein of HPV type 16 (SEQ ID NO: 4; Gly-Gly-Leu-Gly-Ile-Gly-Thr-Gly-Ser-Gly-Thr-Gly-Gly-Arg-Thr-Gly-Tyr-Ile-Pro-Leu) to the N-terminal or the C-terminal of the HBs protein and a chimeric protein obtained by inserting the peptide inside HBs proteins form virus-like particles (VLP) and induce (neutralizing) antibodies against the HBs protein and the peptide. Especially, the present inventors have found that antibodies produced by the chimeric protein wherein the peptide is added to the C-terminal induce antibodies against said peptide early, bind strongly to both the HBs protein and the peptide, and bind to L2 peptide derived from a variety of HPV types, and that antibodies produced by the chimeric protein wherein the peptide is inserted inside HBs protein induce antibodies that recognize the amino acid sequences at two sites in the peptide (N-terminal and C-terminal epitopes) to thereby complete the present invention.

According to the present invention, there are provided a chimeric protein of a peptide comprising an amino acid sequence of 7 to 20 amino acid residues of a specific region of the HPV L2 protein and the HBs protein, a DNA fragment comprising a nucleotide sequence encoding the amino acid sequence of the chimeric protein, an expression vector (including a viral vector) where the DNA fragment is integrated, a host having the expression vector, a vaccine for the prevention of HPV infection and/or hepatitis B using the chimeric proteins and the DNA fragment, and a process for preparing the vaccine.

The chimeric protein and the expression vector comprising the DNA encoding the chimeric protein of the present invention may be effectively used for prevention and treatment of hepatitis B and/or HPV infection. Accordingly, the present invention includes the following:
[1] A chimeric protein of a peptide derived from human papillomavirus (HPV) L2 protein (HPV-L2 peptide) and hepatitis B virus surface protein (HBs protein) wherein the HPV-L2 peptide is (1) a peptide consisting of the core sequence region of 20 amino acid residues, (2) a peptide inside the core sequence region comprising 6 amino acid residues Gly-Gly-Leu-Gly-Ile-Gly or (3) a peptide consisting of 70 or less amino acid residues obtained by adding an amino acid sequence derived from HPV L2 protein at the N-terminal and/or the C-terminal of the core sequence region.
[2] The chimeric protein according to [1] wherein the HPV-L2 peptide consists of a core sequence region.
[3] The chimeric protein according to [1] or [2] wherein the core sequence region is a sequence of Gly-Gly-Leu-Gly-Ile-Gly-Xlaa-Gly-X2aa-Gly-X3aa-Gly-Gly-Arg-X4aa-Gly-Tyr-X5aa-Pro-X6aa wherein X1aa, X2aa, X3aa, X4aa, X5aa and X6aa are an arbitrary amino acid (SEQ ID NO: 3).
[4] The chimeric protein according to [3] wherein the core sequence region is a sequence having the amino acid residues selected from (1) to (6) as follows:
   (1) X1aa is Thr or Ser,
   (2) X2aa is Ser, Thr or Ala,
   (3) X3aa is Thr or Ser,
   (4) X4aa is Ser, Thr or Ala
   (5) X5aa is Ile or Val, and
   (6) X6aa is Leu or Ile
[5] The chimeric protein according to [1] or [2] wherein the core sequence region is the amino acid sequence consisting of Gly-Gly-Leu-Gly-Ile-Gly-Thr-Gly-Ser-Gly-Thr-Gly-Gly-Arg-Thr-Gly-Tyr-Ile-Pro-Leu (SEQ ID NO: 4).
[6] The chimeric protein according to any one of [1] to [5] wherein the chimeric protein contains 1 to 11 HPV-L2 peptides.
[7] The chimeric protein according to any one of [1] to [6] wherein the peptide is added or inserted into at least one of the N-terminal amino acid of the HBs protein, positions 127-128 of SEQ ID NO: 2, or the C-terminal amino acid of the HBs protein.
[8] The chimeric protein according to [7] wherein the peptide is inserted at positions 127-128 of SEQ ID NO: 2 of the HBs protein.
[9] The chimeric protein according to [7] wherein the peptide is added at the C-terminal amino acid of HBs protein.
[10] The chimeric protein according to [7] wherein the peptide is inserted at positions 127-128 of SEQ ID NO: 2 of the HBs protein and added at the C-terminal amino acid.
[11] A chimeric protein of a peptide derived from human papillomavirus (HPV) L2 protein (HPV-L2 peptide) and hepatitis B virus surface protein (HBs protein) wherein the HPV-L2 peptide consists of 20 amino acid residues and has 50-100%, 70-100% or 80-100% sequence homology to the amino acid sequence of Gly-Gly-Leu-Gly-Ile-Gly-Thr-Gly-Ser-Gly-Thr-Gly-Gly-Arg-Thr-Gly-Tyr-Ile-Pro-Leu (SEQ ID NO: 4).
[12] A DNA fragment encoding the chimeric protein as set forth in any one of [1] to [11].
[13] An expression vectors capable of expressing a chimeric protein encoded by the DNA fragment.
[14] A host producing a chimeric protein that is transformed with the expression vector.
[15] A vaccine for HPV infection and/or hepatitis B comprising as an active ingredient the chimeric protein as set forth in any one of [1] to [11].
[16] A vaccine for HPV infection and/or hepatitis B comprising as an active ingredient the chimeric protein as set forth in [8] and the chimeric protein as set forth in [9].
[17] A DNA vaccine for HPV infection and/or hepatitis B comprising as an active ingredient the expression vector as set forth in [13].
[18] A process for preparing a vaccine for human papilloma virus (HPV) infection and/or hepatitis B comprising as an active ingredient a chimeric protein of a peptide derived from HPV L2 protein (HPV-L2 peptide) and hepatitis B virus surface protein (HBs protein), said process comprising the steps (1) to (5) as follows:
   (1) a step of preparing a DNA fragment encoding a chimeric protein of the HBs protein with a HPV-L2 peptide consisting of the core sequence region of 20 amino acid residues, a HPV-L2 peptide inside the core sequence region comprising 6 amino acid residues Gly-Gly-Leu-Gly-Ile-Gly or a HPV-L2 peptide consisting of 70 or less amino acid residues obtained by adding an amino acid sequence derived from HPV L2 protein at the N-terminal and/or the C-terminal of the core sequence region,
   (2) a step of preparing an expression vector comprising the DNA fragment of (1) and transforming a host with the expression vector,
   (3) a step of culturing the transformed host obtained in (2) in the presence of Geneticin and MTX to select the transformed host producing the chimeric protein,
   (4) a step of expanding by culture the transformed host producing the chimeric protein obtained in (3) and purifying the chimeric protein from the culture, and
   (5) a step of adding an adjuvant, a stabilizing agent, a buffer, an isotonic agent and a preservative to the purified chimeric protein obtained in (4) to prepare a vaccine.

### EFFECTS OF THE INVENTION

As one aspect of the present invention, a chimeric protein of the peptide Gly-Gly-Leu-Gly-Ile-Gly-Thr-Gly-Ser-Gly-Thr-Gly-Gly-Arg-Thr-Gly-Tyr-Ile-Pro-Leu with the HBs protein has an ability to produce antibodies against both the peptide and the HBs protein and thus can be a suitable material of a medicament for prophylaxis of HPV infection and/or hepatitis B. In particular, the chimeric protein is useful for increasing the productivity of a vaccine and for improving the quality of a vaccine in that, when the peptide is added to the C-terminal of the HBs protein, not only an increase in the expression level in the host but also an enhanced immune ability of the peptide (early antibody induction) were observed and also, when the peptide is inserted inside the HBs protein, it is possible to induce antibodies against at least the amino acid sequences at two sites in the peptide.

Moreover, the N-terminal 6-amino acid sequence of the peptide is well conserved among strains of HPV and is effective for many HPV types. In fact, as shown in the Examples, antibodies induced by the chimeric protein of the present invention react with many types of HPV. Therefore, according to the present invention, unlike the conventional type-specific vaccines, it is possible to provide a vaccine with a broader spectrum.

### BRIEF DESCRIPTION OF DRAWINGS

Fig. 1 is a graph showing the results of measurement of a concentration of the chimeric proteins in the culture supernatants of transformed cells by sandwich ELISA using anti-HBs monoclonal antibody.
Fig. 2 is a graph showing the reactivity with HPV-L2 peptide derived from the same genotype as the mouse sera (pooled sera) immunized with the chimeric proteins.
Fig. 3 is a graph showing the reactivity with HPV-L2 peptide derived from the same genotype as the mouse sera (individual sera) immunized with the chimeric proteins.
Fig. 4 is a graph showing the reactivity with HPV-L2 peptide derived from the genotype different from the mouse sera (pooled sera) immunized with the chimeric proteins.
Fig. 5 is a graph showing the results of measurement of the HBs antibody titer in mouse sera (pooled sera) immunized with the chimeric proteins.
Fig. 6 is a graph showing the results of epitope analysis of mouse sera (pooled sera) immunized with the chimeric proteins.
Fig. 7 is a graph showing the reactivity with HPV-virus-like particles (VLP) derived from the genotype different from the mouse sera (pooled sera) immunized with the chimeric proteins.
Fig. 8-1 is a graph showing the results of evaluation of the neutralizing ability of the mouse sera (pooled sera) immunized with the chimeric proteins against HPV16-pseudovirus (PsV) of different genotypes.
Fig. 8-2 is a graph showing the results of evaluation of the neutralizing ability of the mouse sera (pooled sera) immunized with the chimeric proteins against HPV18-pseudovirus (PsV) of different genotypes.
Fig. 8-3 is a graph showing the results of evaluation of the neutralizing ability of the mouse sera (pooled sera) immunized with the chimeric proteins against HPV35-pseudovirus (PsV) of different genotypes.

### BEST MODE FOR CARRYING OUT THE INVENTION

The feature of the present invention is that a chimeric protein of a peptide derived from L2 protein of HPV, well conserved among HPV groups, with hepatitis B virus surface protein (HBs protein) is used as an active ingredient of a vaccine for preventing HPV infection and/or hepatitis B.

As described above, HPV, difficult to recover as viral particles, is determined for its genotypes based on the homology of the nucleotide sequence of the capsid (L1) gene, and more than 100 genotypes have been separated so far. For example, types 16, 18, 26, 31, 33, 35, 39, 45, 51, 52, 53, 56, 58, 59, 66, 68, 73 and 82 of HPV are classified as high-risk HPV group whereas types 6 and 11 of HPV are classified as low-risk HPV group. In the L2 protein of HPV is present a region consisting of an amino acid sequence extremely well conserved among high-risk HPV group (hereinafter also referred to as "core sequence region") and the similar core sequence region is also present in low-risk HPV group. A peptides derived from HPV L2 protein consisting of the core sequence region (hereinafter also referred to as "HPV-L2 peptide") is used in the present invention. Such a core sequence region includes 20 amino acids at positions 56-75 for HPV types 16, 31 and 35; 20 amino acids at positions 55-74 for HPV types 39, 45, 51, 52, 56, 58, 66 and 6; 20 amino acids at positions 57-76 for HPV type 73; and 20 amino acids at positions 54-73 for HPV type 11. These 20-amino acid sequences can be shown by the formula: Gly-Gly-Leu-Gly-Ile-Gly-Xlaa-Gly-X2aa-Gly-X3aa-Gly-Gly-Arg-X4aa-Gly-Tyr-X5aa-Pro-X6aa (SEQ ID NO: 3) wherein X1aa, X2aa, X3aa, X4aa, X5aa and X6aa may be an arbitrary amino acid. The arbitrary amino acids in the formula are a site where mutation is observed among the types of HPV strains and are selected as follows:
(1) X1aa is Thr or Ser;
(2) X2aa is Ser, Thr or Ala;
(3) X3aa is Thr or Ser;
(4) X4aa is Ser, Thr or Ala;
(5) X5aa is Ile or Val; and
(6) X6aa is Leu or Ile.

The antibody obtained by immunizing an animal with HPV-L2 peptide consisting of the amino acid sequence: Gly-Gly-Leu-Gly-Ile-Gly-Thr-Gly-Ser-Gly-Thr-Gly-Gly-Arg-Thr-Gly-Tyr-Ile-Pro-Leu (SEQ ID NO: 4) may react with peptides of the core sequence region having a mutation in part. Therefore, it would readily be seen that a common epitope is present in the 6-amino acid sequence (Gly-Gly-Leu-Gly-Ile-Gly) at the N-terminal where no mutation is observed among the types of HPV strains (cf. Example 6). Thus, the HPV-L2 peptide as used in the present invention, in addition to the peptide consisting of the core sequence region of 20 amino acids, also includes a peptide consisting of the 6 amino acids as well as a variety of peptides within the core sequence region wherein amino acids derived from HPV L2 protein are added at the N-terminal and/or the C-terminal of the peptide consisting of the 6 amino acids.

Furthermore, peptides wherein amino acids derived from HPV L2 protein are added at the N-terminal and/or the C-terminal of the core sequence region may also be used in the present invention. HPV-L2 peptides encompass those peptides. Preferably, a total number of amino acids of the HPV-L2 peptide is 70 or less.

Any of HPV-L2 peptides consisting of the core sequence region having the common epitope can be used in the present invention. However, peptides consisting of 20 amino acids having a homology of 50-100%, 70-100% or 80-100% to the amino acid sequence of SEQ ID NO: 4 are preferably used. More preferably, the HPV-L2 peptide shown by the above formula (SEQ ID NO: 3) with amino acid substitutions are used. Most preferable is a HPV-L2 peptide comprising the amino acid sequence of Gly-Gly-Leu-Gly-Ile-Gly-Thr-Gly-Ser-Gly-Thr-Gly-Gly-Arg-Thr-Gly-Tyr-Ile-Pro-Leu (SEQ ID NO: 4).

Furthermore, HPV-12 peptide, as far as it satisfies the conditions described below (retention of the antigenic activity and particle morphology), may encompass the 6 amino acid peptide, 7 to 19 amino acid peptides wherein amino acids derived from HPV L2 protein are added at the N-terminal and/or C-terminal of the 6 amino acids, and peptides wherein amino acids derived from HPV L2 are added at the N-terminal or the C-terminal of the core sequence region consisting of 20 amino acids.

Apart from the above amino acid substitutions, HPV-L2 peptide variants wherein cysteine (Cys) is added to or inserted in HPV-L2 peptide are also one of embodiments of the present invention. In general, it is difficult to obtain a strong immune effect when animals are immunized with a peptide alone and thus a carrier (e.g. KLH) is bound to the peptide or an adjuvant (e.g. Freund's complete adjuvant) is added. Alternatively, addition or insertion of cysteine (Cys) at the N-terminal, the C-terminal, and/or inside of the peptide is carried out in order to improve the immunization ability of the peptide per se (cf. WO 2010/044464 A1 and WO 2011/024748 A1). For example, it is reported that among the amino acid sequence of M2 of influenza virus type A, M2 peptide consisting of 23 amino acids of positions 2-24 wherein a cysteine residue is inserted has a higher immunogenicity (twice to 20-fold) than that of M2 peptide with no modification (cf. WO 2011/024748 A1). If the same effect can be expected by substitution or addition of Cys, one to several amino acids of HPV-L2 peptide of the present invention may be substituted with Cys or Cys may be added provided that the common epitope among HPV groups is conserved. Specifically, since HPV-L2 peptides have exactly the same sequence for the N-terminal 6 amino acids among HPV group, it is preferable to add or insert Cys at any sites other than said amino acid sequence. Hereinafter, if distinction is not necessary between modified and unmodified peptides, these are also simply referred to as HPV-L2 peptide comprehensively.

The sites of addition or insertion of HPV-L2 peptide into the HBs protein may be any sites as far as the immunogenicity against HPV-L2 peptide and HBs protein is retained. Preferably, the chimeric protein of HPV-L2 peptide and HBs protein forms particles and HPV-L2 peptide is located on the surface of the particles. Such a candidate includes the N-terminal, the C-terminal, and a specific site (positions 127-128 of SEQ ID NO: 2) inside the HBs protein. Preferably, HPV-L2 peptide is added at the C-terminal of the HBs protein and/or inserted at the specific site (positions 127-128 of SEQ ID NO: 2) inside the HBs protein.

By adding the peptide at the C-terminal of HBs protein, it is possible to induce antibodies against the peptide early and to retain a higher antibody titer against HBs (Examples 1 and 7). Also, by insertion at a specific site (positions 127-128 of SEQ ID NO: 2) inside the HBs protein, though the antibody induction against HBs decreases, antibodies recognizing amino acid sequences at two sites in the peptide (two epitopes) are induced. This means that antibodies with a broader spectrum for a variety of HPV strains are induced (Example 8). Therefore, in order to induce antibodies against HPV more effectively, HPV-L2 peptide is added at the C-terminal of the HBs protein and is inserted at a specific site (positions 127-128 of SEQ ID NO: 2) inside the HBs protein. The similar effects may be obtained by using a mixture of a chimeric protein obtained by adding HPV-L2 peptide at the C-terminal of HBs protein and a chimeric protein obtained by inserting the peptide at a specific site (positions 127-128 of SEQ ID NO: 2) inside the HBs protein as an immunogen. In this case, induction of a higher antibody titer against the HBs protein is expected. A ratio of the two chimeric proteins for optimal effect may suitably be adjusted by conventional methods.

When adding a foreign peptide at the N-terminal of the HBs protein, it is preferred that the peptide consists of 20 to 70 amino acids. A larger size than this would affect particle formation and a smaller size than this would possibly reduce the antigenic activity. Therefore, HPV-L2 peptide can be used in combination in the present invention as far as the antigenic activity and particle morphology are maintained. In case of HPV-L2 peptide comprising the core sequence region (20 amino acids), two or three peptides connected to each other can be used.

As mentioned above, a peptide consisting of 21 to 70 amino acids in a total length can be used in the present invention that is obtained by adding amino acids from the HPV L2 protein at the N-terminal or the C-terminal of HPV-L2 peptide consisting of the core sequence region. In case of a peptide consisting of 21 to 35 amino acids, two such HPV-L2 peptides can be coupled to each other.

Furthermore, in the case of a peptide consisting of the common epitope sequence (Gly-Gly-Leu-Gly-Ile-Gly) within the core sequence region and peptides consisting of 6-19 amino acids with the common epitope sequence, 2 to 11 of HPV-L2 peptides can be coupled to each other so that the total number of amino acids is 20 to 70 depending on the size of the respective peptides.

Also, as far as the size of peptides added or inserted is in the range of up to 70 amino acids, other peptides in addition to HPV-L2 peptide may be added or inserted. For example, the aforementioned M2 peptide may be added or inserted to prepare a trivalent vaccine effective for HPV infection, hepatitis B and influenza.

A chimeric protein of HPV-L2 peptide and HBs protein of the present invention may be obtained by the following method. First, a gene encoding the HBs protein (HBs gene) is prepared as a basis of a chimeric protein. The nucleotide sequence of the HBs gene has hitherto been disclosed in a number of patent documents, non-patent documents and databases (cf. e.g. Non-Patent reference 10 and Non-Patent reference 11). Therefore, based on the nucleotide sequence of the HBs gene, the gene can easily be obtained by using the common genetic recombination techniques as taught by Sambrook et al. (Molecular Cloning, A Laboratory Manual Second Edition. Cold Spring Harbor Laboratory Press, NY, 1989). For the HBs antigen, any of large, middle and small ones may be used.

Next, a DNA fragment encoding a chimeric protein of HPV-L2 peptide and HBs protein (hereinafter also referred to as "chimeric protein DNA fragment") is prepared by PCR and DNA synthesis. For example, when preparing the chimeric protein DNA fragment wherein HPV-L2 peptide is added at the N-terminal or the C-terminal of the HBs protein by PCR, a primer consisting of a part of the nucleotide sequence encoding HPV-L2 peptide and the HBs gene and the other primer of the HBs gene are used (the direction of the primers is determined based on which primer is at the N-terminal). The similar procedures may be performed when preparing the chimeric protein DNA fragments wherein HPV-L2 peptide is added inside the HBs protein.

PCR primers designed based on the nucleotide sequence encoding HPV-L2 peptide and the nucleotide sequence of the HBs gene are readily available by requesting DNA synthesis trustees (e.g. QIAGEN, Inc.) wherein KOZAK sequence (Kozak M, J.Mol.Biol., 196, 947 (1987)) is added at the 5'-terminal and the nucleotide sequence of appropriate restriction enzyme recognition sites are added at the 5'-terminal and the 3'-terminal depending on the purpose. The DNA fragments amplified by PCR are cloned into cloning vectors such as, for example, pUC119 (TAKARA BIO Inc.), pBlueScript (Agilent Technologies) or pCRII-TOPO (Invitrogen) and sequenced with a DNA sequencer (ABI Prism 377 Applied Biosystems). Confirmation of the chimeric protein DNA fragment of interest is performed by comparing the result of the obtained nucleotide sequence with the sequences of the designed PCR primers and the nucleotide sequence of the existing HBs gene.

The thus obtained chimeric protein DNA fragment of the present invention is incorporated into an appropriate expression vector. The expression vector is introduced into a host for expression of the chimeric protein DNA fragments. For the expression vector, a plasmid, a viral vector and the like can be used. A promoter contained in the expression vector may be selected from promoters such as Lac, tac, pho5, adh, SV40 early, SV40 late, and β-actin, in view of a combination with microorganisms or animal cells to be used as a host. For a host, bacteria, yeast, animal cells, plant cells and insect cells are commonly used and may be selected to suit the intended use. When transforming host cells, known methods may be used. For example, calcium phosphate, DEAE dextran, a method using lipofectin liposome, protoplast polyethylene glycol fusion, electroporation, and the like may be used and appropriately selected so as to suit the host cell used.

In accordance with the present invention, the chimeric protein gene of interest, i.e. the chimeric protein DNA fragments wherein HPV-L2 peptide (20 amino acids at positions 56-75 of SEQ ID NO: 1) is added or inserted at either the N-terminal, the C-terminal or inside (between positions 127-128 of SEQ ID NO: 2) of the HBs protein, was obtained by cleaving the recombinant baculovirus vector (cf. Preparation Example) distributed from National Institute of Infectious Diseases (Dr. Kanda and Dr. Mori) with restriction enzymes. Next, the respective chimeric protein DNA fragments were incorporated into the expression plasmid for animal cell pCAGG-S1(Sal).dhfr.neo (cf. WO2003/004641) and Chinese hamster ovary (CHO) cells were subject to transformation with the resulting expression plasmid by the modified method of calcium phosphate.

Transformed cells expressing the chimeric protein of the present invention are screened in the following two step process. First, the cells subjected to transformation are cultured in a medium containing 10 to 1,000 nmol of L-methotrexate (MTX) and 100 to 1,000 µg/mL of geneticin (Geneticin G418 sulfate) for a given period of time to select and grow the cells that survive so as to select the transformed cells. Then, the cells expressing the chimeric protein are selected among the transformed cells. Specifically, the chimeric proteins of the present invention is confirmed by performing ELISA and WB using the antibody that specifically binds to HPV-L2 peptide or HBs protein on the culture supernatant and cell debris.

For culture of the transformed cells, the conditions as used for culture of normal cells may be used. Thus, T7m medium, Ex-cell SP2/0 medium, YMM-01B medium, YMM-01C medium, OptiCHO medium, Ex-cell 302 medium, or a mixed medium thereof may be used. The medium may be supplemented with fetal calf serum, calcium, magnesium, amino acids, vitamins, and the like. The culture temperature is 32°C to 38°C and the culture period is 2-20 days. In the stage of manufacture of a medicament, it is preferable to maintain the cells expressing the chimeric protein in a serum-free medium.

Purification of the chimeric proteins of the present invention may be achieved by appropriately combining the methods commonly used in protein chemistry such as, for example, centrifugation, salting out, ultrafiltration, isoelectric precipitation, electrophoresis, ion exchange chromatography, gel filtration chromatography, affinity chromatography, hydrophobic chromatography, and hydroxyapatite chromatography. The amount of the resulting chimeric protein may be measured using BCA Protein Assay Reagent Kit (Pierce Biotechnology, Inc), a protein assay kit (Bio-Rad Japan, Inc.), and the like.

The chimeric protein of the present invention may be those obtained by expressing or chemically synthesizing HPV-L2 peptide and HBs protein separately and then chemically conjugating the peptide and the protein. For separate expression, it is possible to conduct amplification by the known gene amplification method using as a template the chemically synthesized DNA sequence or the DNA sequence inserted into a plasmid and to construct the respective expression plasmids by the method described above. The expression plasmid may be introduced into a host as described above to obtain a peptide or a protein of interest.

For chemical conjugation, a method using a crosslinker is exemplified. In that case, an amino group, a thiol group (SH group) and an aldehyde group of a sugar chain present in a protein and a peptide may be used for the conjugation but there is no limitation in the functional groups to be used. Besides, chemical conjugation utilizing the binding with the interaction of biomolecules such as biotin and avidin is also exemplified.

Evaluation of the chimeric protein of the present invention as a vaccine is carried out by immunizing small animals such as chicken, mouse, rat, guinea pig, dog, or monkey with the antigen and then, in in vitro system, after blood collection from the immunized animals, separating the serum and measuring an antibody titer against the chimeric protein of the present invention and a neutralizing antibody titer against HPV and the hepatitis B virus in the serum whereas, in in vivo systems, by administering a pseudo-HPV in the immunized animals and observing survival and the condition. In general, ELISA, PHA and plaque assay are commonly used for the measurement of antibodies in in vitro system. More specifically, a neutralizing antibody against hepatitis B virus may be measured by measuring HBs antibody whereas a neutralizing antibody against HPV may be measured by measuring the binding ability with a virus like particle (VLP) or infectious pseudovirus (Buck CB etc., Efficient intracellular assembly of papillomaviral vectors J Virol 78:. 751-757, 2004).

For immunization procedures (e.g. the site of administration such as subcutaneous, intradermal, intramuscular, intraperitoneal, nasal, oral, and sublingual, and immunization period, etc.), general immunization technique commonly used when examining the immunogenicity of the vaccine may be used. To the antigen used for immunization may be added any adjuvant usable for human for enhancing the immunity, for example, aluminum hydroxide gel, aluminum phosphate gel, CpG oligonucleotides, MDP, QS21, MPL+TDM emulsion, and the like. In addition, various additives acceptable as pharmaceutical applications may also be added for retaining stability and the form of an antigen. Such additives include a stabilizer (arginine, polysorbate 80, macrogol 4000, etc.), an excipient (mannitol, sorbitol, sucrose, and lactose), a buffer (sodium hydrogen phosphate, sodium dihydrogen phosphate, sodium chloride, etc.), a tonicity agent (sodium chloride, etc.), a preservative (thimerosal, phenoxyethanol, etc.), an inactivating agent (formalin, β-propiolactone, etc.), and the like.

The thus obtained chimeric protein of the present invention, having an ability to produce an antibody effective for prevention of HPV infection and growth of hepatitis B, may be used as a material for prophylaxis of HPV infection and hepatitis B or as a material for constructing a detection system of HPV and Hepatitis B virus (e.g. a detection system with antibody measurement such as ELISA, western blotting, and dot blot).

Furthermore, an expression vector wherein the DNA fragment encoding the chimeric protein can express the chimeric protein when administered in vivo may also be used as a DNA vaccine. Such an expression vector includes a plasmid derived from E. coli. For expression of a gene in mammalian cells, the plasmid may include a strong promoter in eukaryotic organisms upstream the gene such as, for example, IE (immediate-early) promoter of cytomegalovirus, β-actin promoter and CAG promoter. Furthermore, by introducing the DNA fragment into viruses and bacteria, a live vaccine may be obtained. Such an expression vector includes viruses (vaccinia virus vector, herpes virus vector, adenovirus vector, Sendai virus vector, measles virus vector, etc.), bacteria (Salmonella vector, etc.), protozoa (malaria vectors, etc.), and the like. For example, when a vaccinia virus vector (LC16m8 strain) in which the DNA fragment encoding the chimeric protein of the present invention is incorporated is used, not only the function as an attenuated live vaccine but also the effect as a smallpox vaccine can be expected. An attenuated live vaccine allows for simplification of a purification process of vaccine material, leading to reduction in cost for the manufacture. A recombinant vaccinia virus in which HBs protein is incorporated expresses particles consisting of only HBs protein (Vaccine 1987 Mar; 5(1) : 65-70). Thus, recombinant vaccinia virus (LC16m8 strain) in which the chimeric protein DNA fragment of the present invention is incorporated is expected to form the particles in the same way and to be an attenuated live vaccine with strong immunogenicity of HPV-L2 peptide being retained.

A vaccine comprising the chimeric protein of the present invention as an active ingredient may be used alone as a bivalent vaccine for preventing HPV infection and hepatitis B or alternatively may be used as a multivalent combined vaccine by combining with at least one vaccine selected from the group consisting of vaccines against infectious diseases of other viruses (e.g. influenza virus, hepatitis A virus, Japanese encephalitis virus, etc.), bacteria (e.g. Haemophilus influenzae, Clostridium tetani, Corynebacterium diphtheria, etc.) and protozoa (e.g. malaria, etc.).

The present invention is explained in more detail by means of the following examples but should not be construed to be limited thereto.

### Preparation Example

### Construction of HPV L2 56/75-HBs chimeric gene: construction of baculovirus expression plasmid

Genes encoding three chimeric proteins in which 20 amino acids at positions 56 to 75 in capsid L2 of HPV type 16 is introduced at the N-terminal, inside between amino acids at positions 127 and 128, or the C-terminal of the HBs and control HBs gene were constructed and introduced into the baculovirus expression plasmid.

### (1) Construction of chimeric gene with N-terminal introduction and construction of baculovirus expression plasmid pFB1-HBsS56/75-N

Using HBs yeast expression plasmid pYG100L (T. Imamura, et al., J. Virol., 61, 3543-3549p, 1987) as a template, PCR amplification was performed with the following Primer NF and Primer R.
Primer NF; 
5'-

gtcgacATGGGTGGGTTAGGAATTGGAACAGGGTCGGGTACAGGCGGACGCACTGGGTA

TATTCCATTGGAGAACACAACATCAGGATT (SEQ ID NO: 18)
 Primer R; 5'-ctgcagTTAAATGTATACCCAAAGAC (SEQ ID NO: 19)

Agarose gel electrophoresis confirmed the band of about 750 bp of the desired size. The band was digested with restriction enzymes SalI-PstI and the digested products were introduced into the restriction enzyme SalI-PstI-digested fragment of baculovirus expression plasmid pFastBacl (Invitrogen) to construct the expression plasmid pFB1-HBsS56/75-N comprising a DNA fragment encoding a chimeric protein (hereinafter also referred to as "56/75-N-terminal introduced chimera") wherein HPV-L2 peptide (20 amino acids at positions 56-75 of SEQ ID NO: 1) was added at the N-terminal of the HBs protein.

### (2) Construction of chimeric gene with introduction between the amino acids at positions 127-128 of HBs and construction of baculovirus expression plasmid pFB1-HBs56/75-127

Using the foregoing pYG100L as a template, PCR amplification was performed with the following Primer F and Primer 127R and with Primer 127F and the aforementioned Primer R to obtain the respective fragments. The respective sequences of Primer F, Primer 127R and Primer 127F are shown below.
Primer F;
5'-gtcgacATGGAGAACACAACATCAGG (SEQ ID NO: 20)
Primer 127R; 5'- 
TCCGCCTGTACCCGACCCTGTTCCAATTCCTAACCCACCAGGAATCGTGCAGGTCTTGC

A (SEQ ID NO: 21)
 Primer 127F;

5'-
CAGGGTCGGGTACAGGCGGACGCACTGGGTATATTCCATTGGCTCAAGGAACCTCTATG
T (SEQ ID NO: 22)

Equal amounts of the respective amplified fragments were mixed together and PCR amplification was further performed with Primer F and Primer R to obtain a gene fragment of about 750 bp wherein the respective fragments were connected to each other. The obtained gene was digested with restriction enzymes Sall-PstI and the digested products were introduced into the restriction enzyme SalI-PstI-digested fragment of the baculovirus expression plasmid pFastBac1 to construct pFB1-HBsS56/75-127 comprising a DNA fragment encoding a chimeric protein (hereinafter also referred to as "56/75-127-position introduced chimera") wherein HPV-L2 peptide (20 amino acids at positions 56-75 of SEQ ID NO: 1) was inserted inside the HBs protein (between positions 127-128 of SEQ ID NO: 2).

### (3) Construction of chimeric gene with C-terminal introduction and construction of baculovirus expression plasmid pFB1-HBsS56/75-N

Using the foregoing pYG100L as a template, PCR amplification was performed with the aforementioned Primer F and the following Primer CR.
Primer CR;

5'-

ctgcagTTACAATGGAATATACCCAGTGCGTCCGCCTGTACCCGACCCTGTTCCAATTC

CTAACCCACCAATGTATACCCAAAGACAAA (SEQ ID NO: 23)

Agarose gel electrophoresis confirmed the band of about 750 bp of the desired size. The band was digested with restriction enzymes SalI-PstI and the digested products were introduced into the restriction enzyme Sall-PstI-digested fragment of baculovirus expression plasmid pFastBacl to construct the expression plasmid pFB1-HBsS56/75-N comprising a DNA fragment encoding a chimeric protein (hereinafter also referred to as "56/75-C-terminal introduced chimera") wherein HPV-L2 peptide (20 amino acids at positions 56-75 of SEQ ID NO: 1) was added at the C-terminal of the HBs protein.

### (4) Construction of HBs gene and construction of baculovirus expression plasmid pFB1-HBsS

For the purpose of introducing the restriction enzyme recognition sites at both terminals of the gene with respect to control HBs gene, PCR amplification was performed with Primer F and Primer R using the aforementioned pYG100L as a template.

Agarose gel electrophoresis confirmed the band of about 690bp of the desired size. The band was digested with restriction enzymes SalI-PstI and the digested products were introduced into the restriction enzyme SalI-PstI-digested fragment of baculovirus expression plasmid pFastBacl to construct the expression plasmid pFB1-HBsS comprising a DNA fragment encoding the HBs protein.

### Example 1

### Construction of animal cell expression plasmid

The expression plasmids (1) to (4) described in Preparation Example were digested with restriction enzymes SalI and XhoI and DNA fragments encoding a chimeric protein of HPV-L2 peptide and HBs protein and a DNA fragment encoding HBs protein were extracted by agarose electrophoresis. The DNA fragments obtained from (1) to (4) are referred to as 56/75-N-terminal introduced chimeric DNA fragment, 56/75-127-position introduced chimeric DNA fragment, 56/75-C-terminal introduced chimeric DNA fragment and HBs DNA fragment, respectively.

Next, each of the above DNA fragments was ligated to animal cell expression plasmid pCAGG-S1(Sal).dhfr.neo (WO2003/004641), previously digested with restriction enzyme SalI and dephosphorylated at the terminals by treatment with calf intestine-derived alkaline phosphatase, to cyclize with Ligation High (Toyobo) to construct 56/75-N-terminal introduced chimeric animal cell expression plasmid (pCAGG.HBs56/75-N.dhfr.neo), 56/75-127-position introduced chimeric animal cell expression plasmid (pCAGG.HBs56/75-127.dhfr.neo), 56/75-C-terminal introduced chimeric animal cell expression plasmid (pCAGG.HBs56/75-C.dhfr.neo) and HBs animal cell expression plasmid (pCAGG.HBs56/75-N.dhfr.neo).

### Example 2

### Expression of chimeric gene using Chinese hamster ovary (CHO) cells

The four animal cell expression plasmids obtained in Example 1 were digested with restriction enzyme PvuI to linearize. CHO Kl (FT Kao et al, Proc Natl Acad Sci USA 60: 1275-1281p, 1968) was transformed with the linearized plasmids by the modified calcium phosphate method (C. Chen et al, Mol. Cell. Biol., 7, 2745-2752p, 1987). After transformation, transformants were selected with dialyzed fetal bovine sera containing 100, 200 or 500 nmol/L of methotrexate (MTX) and 500 µg/mL of geneticin and YMM-01C medium (a self-prepared medium that is prepared by supplementing nucleic acid free MEM alpha medium with amino acids and vitamins and does not contain calcium and magnesium; hereinbelow referred to as "selective medium") supplemented with calcium and magnesium.

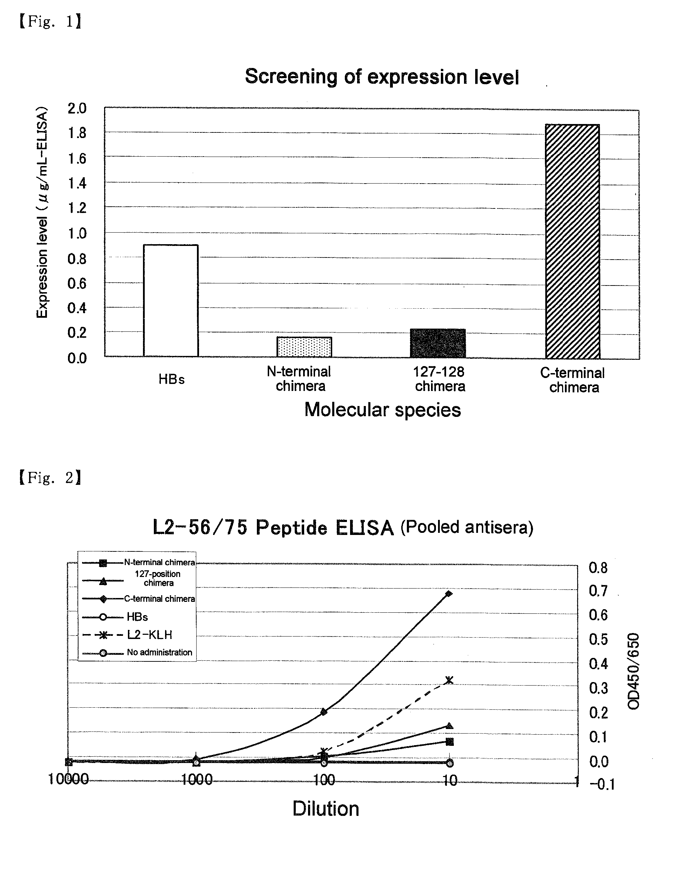

The resulting transformants were released using 0.25% trypsin and expanded using the selection medium described above. After expansion, the transformants were washed with PBS(-) and the medium was exchanged with a serum-free medium (T7m: Ex-cell SP2/0; YMM-01B mixed medium (special order medium manufactured by SAFC Inc.)). After culture at 37°C for 11 days, the concentration of the expressed protein in the culture supernatant was measured by sandwich ELISA using an anti-HBs monoclonal antibody. As a result, 56/75-C-terminal introduced chimera was the highest in the expression level, followed by 56/75-127-position introduced chimera and 56/75-N-terminal introduced chimera. The expression level of 56/75-C-terminal introduced chimera was more than twice higher than that of HBs protein with no introduction as a control (Fig. 1).

### Example 3

### Purification of chimeric protein from cell culture supernatant

The CHO K1 cells expressing the three chimeric proteins and HBs protein obtained in Example 2 were expanded using the selective medium. After expansion, the cells were washed with PBS(-), the medium was exchanged with a serum-free medium (T7m; Ex-cell SP2/0; YMM-01B mixed media) and the cells were cultured at 37°C for more than 10 days. After clarifying the culture by centrifugation, culture supernatant was concentrated with cartridge type UF (Vivacell 100: Sartorius) with an exclusion limit of 1,000 kDa in the presence of Benzonase (Merck). After concentration, the buffer was exchanged with PBS(-), and after addition of cesium chloride at the density of 1.2 g/L, ultracentrifugation was carried out at 4°C, 23,000 x g. The bands containing the chimeric proteins or HBs protein were extracted, and after desalting with UF cartridge with an exclusion limit of 300kDa (Vivaspin; Sartorius) and exchange of the buffer with PBS(-), ultracentrifugation was performed with a discontinuous density gradient of sucrose where PBS(-) containing the chimeric proteins or HBs protein was added to a centrifuge tube overlaid with PBS(-) containing 60%, 50%, 40% or 20% sucrose. After separation by centrifugation under the conditions of 4°C, 120,000 x g, the bands containing the chimeric proteins or HBs protein were extracted, the sugar was removed with UF cartridge with an exclusion limit of 300 kDa, and the buffer was exchanged with PBS(-). Both the resulting chimeric proteins and HBs protein had formed virus-like particles (VPL).

### Example 4

### Immunization of mice with chimeric protein

To the chimeric proteins and HBs protein obtained in Example 3 was added aluminum adjuvant to prepare each 50 µg/ml of immunogen and each 0.1 ml was intramuscularly injected at the thigh of female Balb/c mice (10 animals in each group). After inoculation, antisera were obtained by blood collection at 4 Week (primary immune sera) and also, after inoculation with the same immunogen at 4 Week after the first inoculation, by blood collection at 8 Week (secondary immune sera). As a control, there were used a non-administration group and a group in which an immunogen of a conjugate of the synthetic peptide, obtained by adding Cys to the N-terminal of HPV-L2-derived sequences (SEQ ID NO: 4), and KLH with aluminum as an adjuvant was administered.

### Example 5

### Reactivity of HPV-L2 peptide derived from the same genotype with antisera

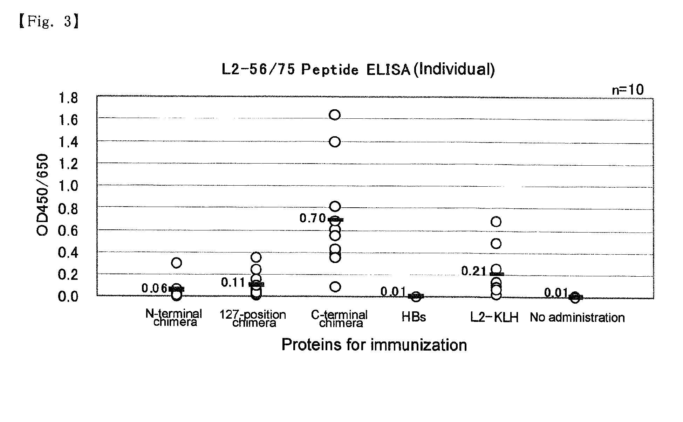

Using individual primary immune sera obtained in Example 4 or a pooled antiserum (a mixture of an equal amount of individual antisera), an ability to produce antibodies against HPV-L2 peptide derived from the same genotype was evaluated by ELISA. More specifically, each well of a 96-well ELISA plate was added with a synthetic peptide (SEQ ID NO: 4)(1.0 µg/well) consisting of 20 amino sequence at positions 56 to 75 of the L2 protein (SEQ ID NO: 1) of HPV type 16 diluted with PBS and was allowed to stand overnight at 4°C. The plate was blocked with a blocking solution (PBS containing 1% BSA and 0.05% Tween20) at room temperature for 2 hours to prepare an antigen immobilized plate. Each serum diluted to 1:100 with the blocking solution was reacted with the immobilized peptide at room temperature for 2 hours. After washing with PBST (PBS containing 0.05% Tween20), horseradish peroxidase (HRP)-labeled anti-mouse IgG antibody (Santa Cruz: sc-2031) diluted to 1:2,000 with the blocking solution was added for reaction at room temperature for 1 hour. After washing with PBST, a substrate solution (TMB+ (DAKO)) was added, and after reaction at room temperature for 10 minutes, absorbance at 450 nm was measured. As a result, antibodies were induced early in the group of immunization with 56/75-C-terminal introduced chimera and the pooled sera showed about 5-fold higher antibody titer than the group of immunization with KLH conjugate (Fig. 2). The average value of the antibody titers obtained in the individual antisera was used for t-test. As a result, significant difference was observed between the two groups (p<0.05; Fig. 3). In addition, antibodies that bind to the synthetic peptide were also observed in the anti-sera of the group of immunization with 56/75-127-position introduced chimera and the group of immunization with 56/75-N-terminal introduced chimera.

### Example 6

### Reactivity of HPV-L2 peptide derived from the different genotype with antisera

Using individual primary immune sera obtained in Example 4 or a pooled antiserum (a mixture of an equal amount of individual antisera), an ability to bind to HPV-L2 peptide in the common area derived from different genotypes was evaluated using the procedures described in Example 5 (HPV type 18, HPV type 59 and HPV type 68 have the same sequence as HPV type 16). The synthetic peptides of the following amino acid sequences were used. Note that in the following amino acid sequences, asterisk (*) indicates the same amino acid as that of the HPV16 type.
High-risk type
HPV16-L2-56/75 56-GGLGIGTGSGTGGRTGYIPL (SEQ ID NO: 4)
HPV31-L2-56/75 56-******S**********V**
(2 amino acid substitutions) (SEQ ID NO: 5)
HPV33-L2-55/74 55-**********S******V*I
(3 amino acid substitutions) (SEQ ID NO: 6)
HPV35-L2-56/75 56-******S*******S**V**
(3 amino acid substitutions) (SEQ ID NO: 7)
HPV39-L2-55/74 55-********T***********
(1 amino acid substitution) (SEQ ID NO: 8)
HPV45-L2-55/74 55-**********S******V**
(2 amino acid substitutions) (SEQ ID NO: 9)
HPV51-L2-55/74 55-**********S*********
(1 amino acid substitution) (SEQ ID NO: 10)
HPV52-L2-55/74 55-********A*S***A**V**
(4 amino acid substitutions) (SEQ ID NO: 11)
HPV56-L2-55/74 55-********T*S***A**V**
(4 amino acid substitutions) (SEQ ID NO: 12)
HPV58-L2-55/74 55-*****************V**
(1 amino acid substitution) (SEQ ID NO: 13)
HPV66-L2-55/74 55-**********S***A**V**
(3 amino acid substitutions) (SEQ ID NO: 14) HPV73-L2-57/76 57-******S***S******V**
(3 amino acid substitutions) (SEQ ID NO: 15)
Twelve peptides in total
Low-risk type
HPV6-L2-55/74 55-*****************V**
(1 amino acid substitution) (SEQ ID NO: 16)
HPV11-L2-54/73 54-********A*S***A*****
(3 amino acid substitutions) (SEQ ID NO: 17)
Two peptides in total

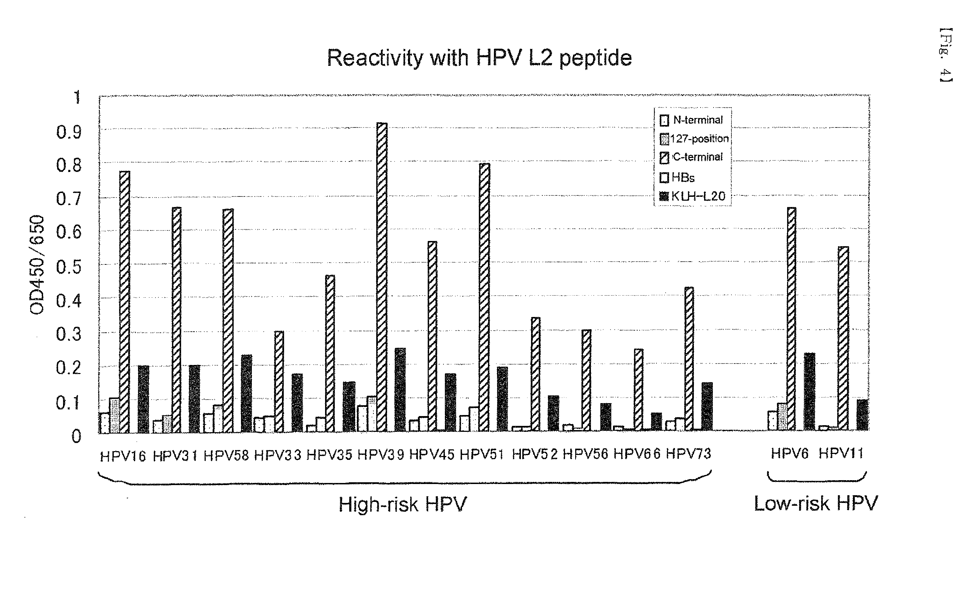

It was confirmed that antisera of the groups of immunization with the chimeric proteins reacted with all of the synthetic peptides described above. In particular, the group of immunization with 56/75-C-terminal introduced chimera showed a higher antibody titer as compared to the other groups (Fig. 4).

### Example 7

### Measurement of HBs antibody titer in anti-sera

For the pooled antiserum which is a mixture of equal amounts of the individual primary immune sera obtained in Example 4, an antibody titer against HBs protein was measured. The pooled antisera were subjected to 10-fold serial dilution with PBS(-) (10-, 100- and 1,000-folds) and an antibody titer was measured with the automatic enzyme immunoassay equipment AIA-360 using hepatitis B virus surface antibody kit E test "TOSOH" II (HBsAb; Tosoh Corporation).

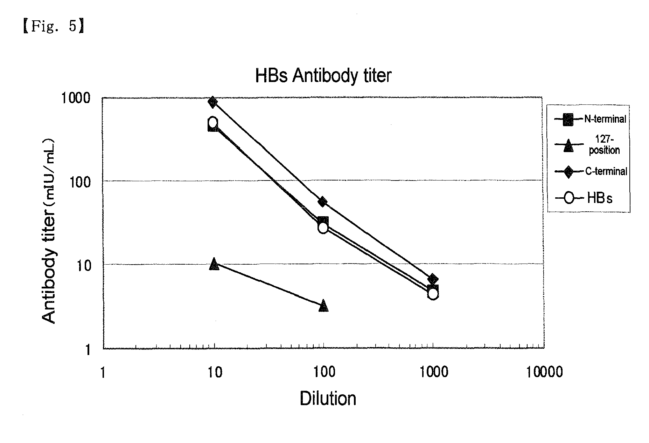

In any of the groups of immunization with the chimeric proteins, antibodies reactive to HBs protein were detected. A high antibody titer was obtained for the group of immunization with 56/75-N-terminal introduced chimera and the group of immunization with 56/75-C-terminal introduced chimera as in the group of immunization with HBs protein alone. However, an antibody titer increase was suppressed to about 1/10 for the group of immunization with 56/75-127-position introduced chimera (Fig. 5).

### Example 8

### Epitope analysis of antisera

An ability to bind to HPV-L2 peptide was evaluated for a pooled antiserum, a mixture of an equal amount of individual secondary immune sera obtained in Example 4, by ELISA using the synthetic peptides of the following amino acid sequences. Specifically, mutated peptides of HPV16 L2 with deletion or alanine substitution in amino acids at positions 53 to 75 of HPV16 L2 were coupled to BSA via cysteine added at the N-terminal for use as an antigen. Each well of 96-well ELISA plate was added with BSA-bound peptides (500 ng/well) diluted in PBS and was allowed to stand overnight at 4°C. The plate was blocked with a blocking solution (PBS containing 5% skim milk and 0.1% Tween20) at room temperature for 2 hours to prepare an antigen immobilized plate. Each serum was subjected to serial dilution from 1:100 to 1:2,500 with the blocking solution and reacted with the immobilized peptide at room temperature for 2 hours. After washing with PBST (PBS containing 0.1% Tween20), horseradish peroxidase (HRP)-labeled anti-mouse IgG antibody (Santa Cruz: sc-2031) diluted to 1:2,000 with the blocking solution was added for reaction at room temperature for 1 hour. After washing with PBST, a substrate solution (0.1M trisodium citrate containing 2 mg/ml of o-phenylenediamine and 0.0065% hydrogen peroxide, pH4.8) was added, and after reaction at room temperature for 10 minutes, absorbance at 450 nm was measured.
HPV16-L2-14/27 14-SATQLYKTCKQAGT (SEQ ID NO: 24)
HPV16-L2-53/73 53-VFFGGLGIGTGSGTGGRTGYI (SEQ ID NO: 25)
HPV16-L2-53/70 53-VFFGGLGIGTGSGTGGRTAAA (SEQ ID NO: 26)
HPV16-L2-53/67 53-VFFGGLGIGTGSGTGAAAAAA (SEQ ID NO: 27)
HPV16-L2-53/64 53-VFFGGLGIGTGSAAAAAAAAA (SEQ ID NO: 28)
HPV16-L2-55/75 55-FGGLGIGTGSGTGGRTGYIPL (SEQ ID NO: 29)
HPV16-L2-58/75 58-AAALGIGTGSGTGGRTGYIPL (SEQ ID NO: 30)
HPV16-L2-60/75 60-AAAAAAGTGSGTGGRTGYIPL (SEQ ID NO: 31)
HPV16-L2-64/75 64-AAAAAAAAASGTGGRTGYIPL (SEQ ID NO: 32)

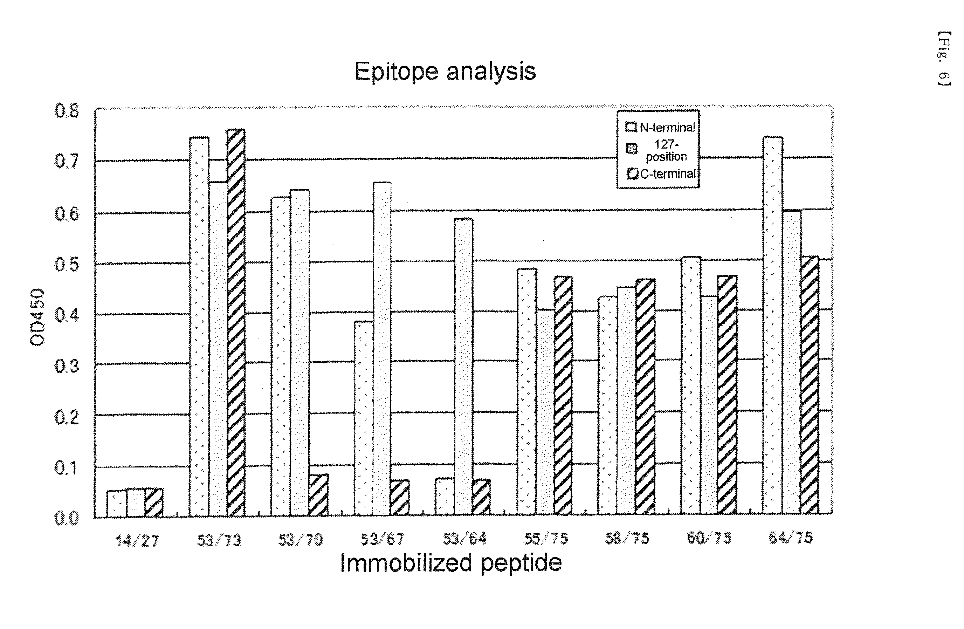

As a result, all of the immunization groups had a high antibody titer. It was shown that antibodies recognizing two amino acid sequences in the N-terminal and C-terminal of the core sequence region (20 amino acids) (N-terminal and C-terminal epitopes) were induced for the group of immunization with 56/75-127-position introduced chimera whereas antibodies recognizing the C-terminal amino acid sequence (C-terminal epitope) were induced for the group of immunization with 56/75-N-terminal introduced chimera and the group of immunization with 56/75-C-terminal introduced chimera (Fig. 6).

### Example 9

### Reactivity of HPV-virus-like particle (VLP) from different genotypes with antisera

An ability to bind to HPV-L2 peptide was evaluated for a pooled antiserum, a mixture of an equal amount of individual secondary immune sera (the group of immunization with 56/75-127-position introduced chimera), obtained in Example 4 by the procedures described below.

### (1) Preparation of VLP

293FT Cells (Invitrogen) were transfected with L1 and L2 expression plasmid of HPV type 16, 18, 31, 33, 35, 51, 52 or 58. After 2 days, the cells were dissolved in Lysis Buffer (PBS containing 0.35% Brij58, 0.9 mM CaCl₂, 10 mM MgCl₂ and 1 µl Benzonase) and incubated overnight at 37°C. 5M NaCl was added to a final concentration of 0.8M and, after being left to stand on ice for 10 minutes, the solution was centrifuged at 10,000g, 4°C for 10 minutes, and the supernatant was recovered. The supernatant was layered on top of 27%, 33% and 39% of iodixanol (adjusted with 0.8M NaCl in PBS) and subjected to ultracentrifugation at 50,000 rpm, 16°C for 3.5 hours. After ultracentrifugation, fractions containing the VLP were collected.

### (2) ELISA

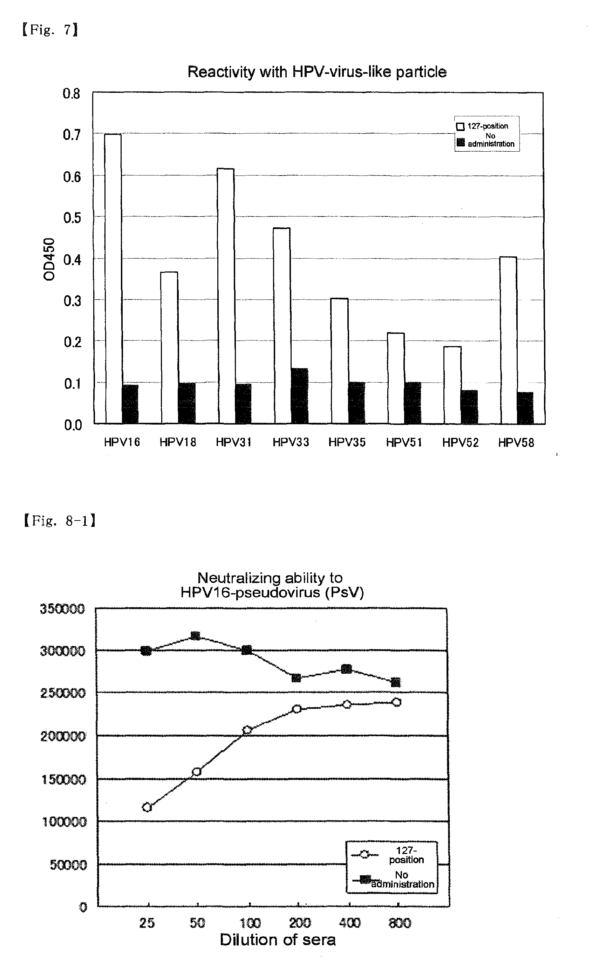

Each well of 96-well ELISA plate was added with VLP (100 ng/well) diluted in PBS and was allowed to stand overnight at 4°C. The plate was blocked with a blocking solution (PBS containing 5% skim milk and 0.1% Tween20) at room temperature for 2 hours to prepare an antigen immobilized plate. Each serum was subjected to serial dilution from 1:20 to 1:20,000 with the blocking solution and reacted with the immobilized VLP at room temperature for 2 hours. Then, in the same manner as in ELISA for the peptide antigen, antibodies bound to VLP were detected. As a result, the antibodies bound with all of the HPV-VLP to prove to have a broad spectrum against HPV (Fig. 7).

### Example 10

### Reactivity of HPV-pseudovirus (PsV) from different genotypes with antisera

The neutralizing ability to HPV-PsV was evaluated for a pooled antiserum, a mixture of an equal amount of individual secondary immune sera (the group of immunization with 56/75-127-position introduced chimera), obtained in Example 4 by the procedures described below.

### (1) Preparation of PsV

L1 and L2 expression plasmid of HPV type 16, 18 or 35 and secreted alkaline phosphatase (SEAP) expression plasmid were mixed and 293FT Cells were transfected with the mixture. After 2 days, PsV was purified in the same manner as VLP.

### (2) Neutralization test

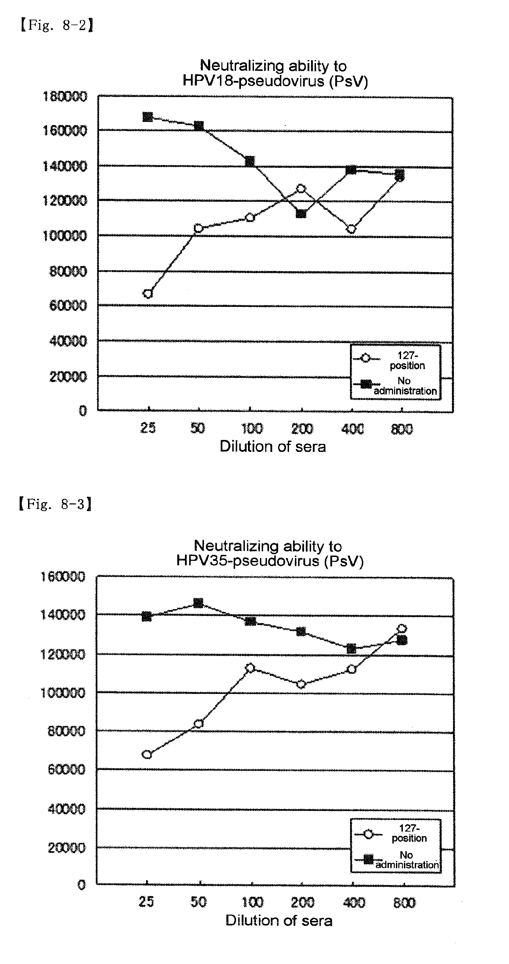

Equal amounts of PsV diluted in medium for cell culture (phenol red-free DMEM containing 10% FBS) and of each serum serially diluted with the same medium from 1:12.5 to 1:400 were mixed and allowed to react at 4°C for 1 hour, followed by infection to 293FT cells previously prepared in 96-well plate (1 × 10⁴ cells/well). After 3 days, the SEAP activity in the culture supernatant was measured using Great EscAPe SEAP Assay Kit (Clontech) and a plate reader (ARVO MX, PerkinElmer). As a result, the neutralizing activity was definitely observed for any of the pseudovirus (Fig. 8-1 to Fig. 8-3).

### INDUSTRIAL APPLICABILITY

The chimeric proteins of the present invention may be utilized in the material of prophylactics for human papillomavirus (HPV) infection and hepatitis B.

## Claims

1. A chimeric protein of a peptide derived from human papillomavirus (HPV) L2 protein (HPV-L2 peptide) and hepatitis B virus surface protein (HBs protein) wherein the HPV-L2 peptide is (1) a peptide consisting of the core sequence region of 20 amino acid residues, (2) a peptide inside the core sequence region comprising 6 amino acid residues Gly-Gly-Leu-Gly-Ile-Gly or (3) a peptide consisting of 70 or less amino acid residues obtained by adding an amino acid sequence derived from HPV L2 protein at the N-terminal and/or the C-terminal of the core sequence region.

2. The chimeric protein according to claim 1 wherein the HPV-L2 peptide consists of a core sequence region.

3. The chimeric protein according to claim 1 or 2 wherein the core sequence region is a sequence of Gly-Gly-Leu-Gly-Ile-Gly-Xlaa-Gly-X2aa-Gly-X3aa-Gly-Gly-Arg-X4aa-Gly-Tyr-X5aa-Pro-X6aa wherein X1aa, X2aa, X3aa, X4aa, X5aa and X6aa are an arbitrary amino acid (SEQ ID NO: 3).

4. The chimeric protein according to claim 3 wherein the core sequence region is a sequence having the amino acid residues selected from (1) to (6) as follows:
(1) X1aa is Thr or Ser,
(2) X2aa is Ser, Thr or Ala,
(3) X3aa is Thr or Ser,
(4) X4aa is Ser, Thr or Ala
(5) X5aa is Ile or Val, and
(6) X6aa is Leu or Ile

5. The chimeric protein according to claim 1 or 2 wherein the core sequence region is the amino acid sequence consisting of Gly-Gly-Leu-Gly-Ile-Gly-Thr-Gly-Ser-Gly-Thr-Gly-Gly-Arg-Thr-Gly-Tyr-Ile-Pro-Leu (SEQ ID NO: 4).

6. The chimeric protein according to any one of claims 1 to 5 wherein the chimeric protein contains 1 to 11 HPV-L2 peptides.

7. The chimeric protein according to any one of claims 1 to 6 wherein the peptide is added or inserted into at least one of the N-terminal amino acid of the HBs protein, positions 127-128 of SEQ ID NO: 2, or the C-terminal amino acid of the HBs protein.

8. The chimeric protein according to claim 7 wherein the peptide is inserted at positions 127-128 of SEQ ID NO: 2 of the HBs protein.

9. The chimeric protein according to claim 7 wherein the peptide is added at the C-terminal amino acid of HBs protein.

10. The chimeric protein according to claim 7 wherein the peptide is inserted at positions 127-128 of SEQ ID NO: 2 of the HBs protein and added at the C-terminal amino acid.

11. A chimeric protein of a peptide derived from human papillomavirus (HPV) L2 protein (HPV-L2 peptide) and hepatitis B virus surface protein (HBs protein) wherein the HPV-L2 peptide consists of 20 amino acid residues and has 50-100%, 70-100% or 80-100% sequence homology to the amino acid sequence of Gly-Gly-Leu-Gly-Ile-Gly-Thr-Gly-Ser-Gly-Thr-Gly-Gly-Arg-Thr-Gly-Tyr-Ile-Pro-Leu (SEQ ID NO: 4).

12. A DNA fragment encoding the chimeric protein as set forth in any one of claims 1 to 11.

13. An expression vectors capable of expressing a chimeric protein encoded by the DNA fragment.

14. A host producing a chimeric protein that is transformed with the expression vector.

15. A vaccine for HPV infection and/or hepatitis B comprising as an active ingredient the chimeric protein as set forth in any one of claims 1 to 11.

16. A vaccine for HPV infection and/or hepatitis B comprising as an active ingredient the chimeric protein as set forth in claim 8 and the chimeric protein as set forth in claim 9.

17. A DNA vaccine for HPV infection and/or hepatitis B comprising as an active ingredient the expression vector as set forth in claim 13.

18. A process for preparing a vaccine for human papilloma virus (HPV) infection and/or hepatitis B comprising as an active ingredient a chimeric protein of a peptide derived from HPV L2 protein (HPV-L2 peptide) and hepatitis B virus surface protein (HBs protein), said process comprising the steps (1) to (5) as follows:
(1) a step of preparing a DNA fragment encoding a chimeric protein of the HBs protein with a HPV-L2 peptide consisting of the core sequence region of 20 amino acid residues, a HPV-L2 peptide inside the core sequence region comprising 6 amino acid residues Gly-Gly-Leu-Gly-Ile-Gly or a HPV-L2 peptide consisting of 70 or less amino acid residues obtained by adding an amino acid sequence derived from HPV L2 protein at the N-terminal and/or the C-terminal of the core sequence region,
(2) a step of preparing an expression vector comprising the DNA fragment of (1) and transforming a host with the expression vector,
(3) a step of culturing the transformed host obtained in (2) in the presence of Geneticin and MTX to select the transformed host producing the chimeric protein,
(4) a step of expanding by culture the transformed host producing the chimeric protein obtained in (3) and purifying the chimeric protein from the culture, and
(5) a step of adding an adjuvant, a stabilizing agent, a buffer, an isotonic agent and a preservative to the purified chimeric protein obtained in (4) to prepare a vaccine.


SEQUENCE LISTING

<110>  THE CHEMO-SERO-THERAPEUTIC RESEARCH INSTITUTE

<120>  A vaccine against HPV infection and/or Hepatitis B containing an
       HPV/HBs chimera protein as active ingredient

<130>  671762

<150>  JP 2012-280850
<151>  2012-12-25

<160>  32

<170>  PatentIn version 3.4

<210>  1
<211>  473
<212>  PRT
<213>  human papillomavirus

<400>  1

Met Arg His Lys Arg Ser Ala Lys Arg Thr Lys Arg Ala Ser Ala Thr
1               5                   10                  15

Gln Leu Tyr Lys Thr Cys Lys Gln Ala Gly Thr Cys Pro Pro Asp Ile
            20                  25                  30

Ile Pro Lys Val Glu Gly Lys Thr Ile Ala Glu Gln Ile Leu Gln Tyr
        35                  40                  45

Gly Ser Met Gly Val Phe Phe Gly Gly Leu Gly Ile Gly Thr Gly Ser
    50                  55                  60

Gly Thr Gly Gly Arg Thr Gly Tyr Ile Pro Leu Gly Thr Arg Pro Pro
65                  70                  75                  80

Thr Ala Thr Asp Thr Leu Ala Pro Val Arg Pro Pro Leu Thr Val Asp
                85                  90                  95

Pro Val Gly Pro Ser Asp Pro Ser Ile Val Ser Leu Val Glu Glu Thr
            100                 105                 110

Ser Phe Ile Asp Ala Gly Ala Pro Thr Ser Val Pro Ser Ile Pro Pro
        115                 120                 125

Asp Val Ser Gly Phe Ser Ile Thr Thr Ser Thr Asp Thr Thr Pro Ala
    130                 135                 140

Ile Leu Asp Ile Asn Asn Thr Val Thr Thr Val Thr Thr His Asn Asn
145                 150                 155                 160

Pro Thr Phe Thr Asp Pro Ser Val Leu Gln Pro Pro Thr Pro Ala Glu
                165                 170                 175

Thr Gly Gly His Phe Thr Leu Ser Ser Ser Thr Ile Ser Thr His Asn
            180                 185                 190

Tyr Glu Glu Ile Pro Met Asp Thr Phe Ile Val Ser Thr Asn Pro Asn
        195                 200                 205

Thr Val Thr Ser Ser Thr Pro Ile Pro Gly Ser Arg Pro Val Ala Arg
    210                 215                 220

Leu Gly Leu Tyr Ser Arg Thr Thr Gln Gln Val Lys Val Val Asp Pro
225                 230                 235                 240

Ala Phe Val Thr Thr Pro Thr Lys Leu Ile Thr Tyr Asp Asn Pro Ala
                245                 250                 255

Tyr Glu Gly Ile Asp Val Asp Asn Thr Leu Tyr Phe Ser Ser Asn Asp
            260                 265                 270

Asn Ser Ile Asn Ile Ala Pro Asp Pro Asp Phe Leu Asp Ile Val Ala
        275                 280                 285

Leu His Arg Pro Ala Leu Thr Ser Arg Arg Thr Gly Ile Arg Tyr Ser
    290                 295                 300

Arg Ile Gly Asn Lys Gln Thr Leu Arg Thr Arg Ser Gly Lys Ser Ile
305                 310                 315                 320

Gly Ala Lys Val His Tyr Tyr Tyr Asp Leu Ser Thr Ile Asp Pro Ala
                325                 330                 335

Glu Glu Ile Glu Leu Gln Thr Ile Thr Pro Ser Thr Tyr Thr Thr Thr
            340                 345                 350

Ser His Ala Ala Ser Pro Thr Ser Ile Asn Asn Gly Leu Tyr Asp Ile
        355                 360                 365

Tyr Ala Asp Asp Phe Ile Thr Asp Thr Ser Thr Thr Pro Val Pro Ser
    370                 375                 380

Val Pro Ser Thr Ser Leu Ser Gly Tyr Ile Pro Ala Asn Thr Thr Ile
385                 390                 395                 400

Pro Phe Gly Gly Ala Tyr Asn Ile Pro Leu Val Ser Gly Pro Asp Ile
                405                 410                 415

Pro Ile Asn Ile Thr Asp Gln Ala Pro Ser Leu Ile Pro Ile Val Pro
            420                 425                 430

Gly Ser Pro Gln Tyr Thr Ile Ile Ala Asp Ala Gly Asp Phe Tyr Leu
        435                 440                 445

His Pro Ser Tyr Tyr Met Leu Arg Lys Arg Arg Lys Arg Leu Pro Tyr
    450                 455                 460

Phe Phe Ser Asp Val Ser Leu Ala Ala
465                 470

<210> 2
<211> 226
<212> PRT
<213> Hepatitis B virus

<400> 2

Met Glu Asn Thr Thr Ser Gly Phe Leu Gly Pro Leu Leu Val Leu Gln
1               5                   10                  15

Ala Gly Phe Phe Leu Leu Thr Arg Ile Leu Thr Ile Pro Gln Ser Leu
            20                  25                  30

Asp Ser Trp Trp Thr Ser Leu Asn Phe Leu Gly Gly Ala Pro Thr Cys
        35                  40                  45

Pro Gly Gln Asn Ser Gln Ser Pro Thr Ser Asn His Ser Pro Thr Ser
    50                  55                  60

Cys Pro Pro Ile Cys Pro Gly Tyr Arg Trp Met Cys Leu Arg Arg Phe
65                  70                  75                  80

Ile Ile Phe Leu Phe Ile Leu Leu Leu Cys Leu Ile Phe Leu Leu Val
                85                  90                  95

Leu Leu Asp Tyr Gln Gly Met Leu Pro Val Cys Pro Leu Leu Pro Gly
            100                 105                 110

Thr Ser Thr Thr Ser Thr Gly Pro Cys Lys Thr Cys Thr Ile Pro Ala
        115                 120                 125

Gln Gly Thr Ser Met Phe Pro Ser Cys Cys Cys Thr Lys Pro Ser Asp
    130                 135                 140

Gly Asn Cys Thr Cys Ile Pro Ile Pro Ser Ser Trp Ala Phe Ala Arg
145                 150                 155                 160

Phe Leu Trp Glu Trp Ala Ser Val Arg Phe Ser Trp Leu Ser Leu Leu
                165                 170                 175

Val Pro Phe Val Gln Trp Phe Val Gly Leu Ser Pro Thr Val Trp Leu
            180                 185                 190

Ser Val Ile Trp Met Met Trp Tyr Trp Gly Pro Ser Leu Tyr Asn Ile
        195                 200                 205

Leu Ser Pro Phe Leu Pro Leu Leu Pro Ile Phe Phe Cys Leu Trp Val
    210                 215                 220

Tyr Ile
225

<210>  3
<211>  20
<212>  PRT
<213>  human papillomavirus

<220>
<221>  misc_feature
<222>  (7)..(7)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (11)..(11)
<223>  Xaa can be any naturally occurring amino acid

<220>
<221>  misc_feature
<222>  (15)..(15)
<223>  Xaa can be any naturally occurring amino acid

<220>
<221>  misc_feature
<222>  (18)..(18)
<223>  Xaa can be any naturally occurring amino acid

<220>
<221>  misc_feature
<222>  (20)..(20)
<223>  Xaa can be any naturally occurring amino acid

<400>  3

Gly Gly Leu Gly Ile Gly Xaa Gly Xaa Gly Xaa Gly Gly Arg Xaa Gly
1               5                   10                  15

Tyr Xaa Pro Xaa
            20

<210>  4
<211>  20
<212>  PRT
<213>  human papillomavirus

<400>  4

Gly Gly Leu Gly Ile Gly Thr Gly Ser Gly Thr Gly Gly Arg Thr Gly
1               5                   10                  15

Tyr Ile Pro Leu
            20

<210>  5
<211>  20
<212>  PRT
<213>  human papillomavirus

<400>  5

Gly Gly Leu Gly Ile Gly Ser Gly Ser Gly Thr Gly Gly Arg Thr Gly
1               5                   10                  15

Tyr Val Pro Leu
            20

<210>  6
<211>  20
<212>  PRT
<213>  human papillomavirus

<400>  6

Gly Gly Leu Gly Ile Gly Thr Gly Ser Gly Ser Gly Gly Arg Thr Gly
1               5                   10                  15

Tyr Val Pro Ile
            20

<210>  7
<211>  20
<212>  PRT
<213>  human papillomavirus

<400>  7

Gly Gly Leu Gly Ile Gly Ser Gly Ser Gly Thr Gly Gly Arg Ser Gly
1               5                   10                  15

Tyr Val Pro Leu
            20

<210>  8
<211>  19
<212>  PRT
<213>  human papillomavirus

<400>  8

Gly Gly Leu Gly Ile Gly Thr Gly Gly Thr Gly Gly Arg Thr Gly Tyr
1               5                   10                  15

Ile Pro Leu

<210>  9
<211>  20
<212>  PRT
<213>  human papillomavirus

<400>  9

Gly Gly Leu Gly Ile Gly Thr Gly Ser Gly Ser Gly Gly Arg Thr Gly
1               5                   10                  15

Tyr Val Pro Leu
            20

<210>  10
<211>  20
<212>  PRT
<213>  human papillomavirus

<400>  10

Gly Gly Leu Gly Ile Gly Thr Gly Ser Gly Ser Gly Gly Arg Thr Gly
1               5                   10                  15

Tyr Ile Pro Leu
            20

<210>  11
<211>  20
<212>  PRT
<213>  human papillomavirus

<400>  11

Gly Gly Leu Gly Ile Gly Thr Gly Ala Gly Ser Gly Gly Arg Ala Gly
1               5                   10                  15

Tyr Val Pro Leu
            20

<210>  12
<211>  20

<212>  PRT
<213>  human papillomavirus

<400>  12

Gly Gly Leu Gly Ile Gly Thr Gly Thr Gly Ser Gly Gly Arg Ala Gly
1               5                   10                  15

Tyr Val Pro Leu
            20

<210>  13
<211>  20
<212>  PRT
<213>  human papillomavirus

<400>  13

Gly Gly Leu Gly Ile Gly Thr Gly Ser Gly Thr Gly Gly Arg Thr Gly
1               5                   10                  15

Tyr Val Pro Leu
            20

<210>  14
<211>  20
<212>  PRT
<213>  human papillomavirus

<400>  14

Gly Gly Leu Gly Ile Gly Thr Gly Ser Gly Ser Gly Gly Arg Ala Gly
1               5                   10                  15

Tyr Val Pro Leu
            20

<210>  15
<211>  20
<212>  PRT
<213>  human papillomavirus

<400>  15

Gly Gly Leu Gly Ile Gly Ser Gly Ser Gly Ser Gly Gly Arg Thr Gly
1               5                   10                  15

Tyr Val Pro Leu
            20

<210>  16
<211>  20
<212>  PRT
<213>  human papillomavirus

<400>  16

Gly Gly Leu Gly Ile Gly Thr Gly Ser Gly Thr Gly Gly Arg Thr Gly
1               5                   10                  15

Tyr Val Pro Leu
            20

<210>  17
<211>  20
<212>  PRT
<213>  human papillomavirus

<400>  17

Gly Gly Leu Gly Ile Gly Thr Gly Ala Gly Ser Gly Gly Arg Ala Gly
1               5                   10                  15

Tyr Ile Pro Leu
            20

<210>  18
<211>  89
<212>  DNA
<213>  Artificial

<220>
<223>  5' Primer to obtain a chimera protein that HPV L2 peptide is
       added to the N-terminal site of HBs protein by PCR.

<400>  18
gtcgacatgg gtgggttagg aattggaaca gggtcgggta caggcggacg cactgggtat      60

attccattgg agaacacaac atcaggatt                                        89

<210>  19
<211>  26
<212>  DNA
<213>  Artificial

<220>
<223>  3' Primer to obtain a chimera protein that HPV L2 peptide is
       added to the N-terminal site of HBs protein by PCR.

<400>  19
ctgcagttaa atgtataccc aaagac                                           26

<210>  20
<211>  26
<212>  DNA
<213>  Artificial

<220>
<223>  5' Primer to obtain a chimera protein that HPV L2 peptide is
       added to the C-terminal site of the peptide consisting of the
       N-terminal half of HBs protein by PCR.

<400>  20
gtcgacatgg agaacacaac atcagg                                          26


<210>  21
<211>  60
<212>  DNA
<213>  Artificial

<220>
<223>  3' Primer to obtain a chimera protein that HPV L2 peptide is
       added to the C-terminal site of the peptide consisting of the
       N-terminal half of HBs protein by PCR.

<400>  21
tccgcctgta cccgaccctg ttccaattcc taacccacca ggaatcgtgc aggtcttgca     60


<210>  22
<211>  60
<212>  DNA
<213>  Artificial

<220>
<223>  5' Primer to obtain a chimera protein that HPV L2 peptide is
       added to the N-terminal site of the peptide consisting of the
       C-terminal half of HBs protein by PCR.

<400>  22
cagggtcggg tacaggcgga cgcactgggt atattccatt ggctcaagga acctctatgt     60


<210>  23
<211>  89
<212>  DNA
<213>  Artificial

<220>
<223>  3' Primer to obtain a chimera protein that HPV L2 peptide is
       added to the C-terminal site of HBs protein by PCR.

<400>  23
ctgcagttac aatggaatat acccagtgcg tccgcctgta cccgaccctg ttccaattcc     60

taacccacca atgtataccc aaagacaaa                                       89


<210>  24
<211>  14
<212>  PRT
<213>  Human papillomavirus

<400>  24

Ser Ala Thr Gln Leu Tyr Lys Thr Cys Lys Gln Ala Gly Thr
1               5                   10


<210>  25
<211>  21
<212>  PRT
<213>  Human papillomavirus

<400>  25

Val Phe Phe Gly Gly Leu Gly Ile Gly Thr Gly Ser Gly Thr Gly Gly
1               5                   10                  15

Arg Thr Gly Tyr Ile
            20

<210>  26
<211>  21
<212>  PRT
<213>  Artificial

<220>
<223>  A HPV L2-derived peptide to which three alanines are added at its
       C-terminal.

<400>  26

Val Phe Phe Gly Gly Leu Gly Ile Gly Thr Gly Ser Gly Thr Gly Gly
1               5                   10                  15

Arg Thr Ala Ala Ala
            20

<210>  27
<211>  21
<212>  PRT
<213>  Artificial

<220>
<223>  A HPV L2-derived peptide to which six alanines are added at its
       C-terminal.

<400>  27

Val Phe Phe Gly Gly Leu Gly Ile Gly Thr Gly Ser Gly Thr Gly Ala
1               5                   10                  15

Ala Ala Ala Ala Ala
            20

<210>  28
<211>  21
<212>  PRT
<213>  Artificial

<220>
<223>  A HPV L2-derived peptide to which nine alanines are added at its
       C-terminal.

<400>  28

Val Phe Phe Gly Gly Leu Gly Ile Gly Thr Gly Ser Ala Ala Ala Ala
1               5                   10                  15

Ala Ala Ala Ala Ala
            20

<210>  29
<211>  21
<212>  PRT
<213>  Human papillomavirus

<400>  29

Phe Gly Gly Leu Gly Ile Gly Thr Gly Ser Gly Thr Gly Gly Arg Thr
1               5                   10                  15

Gly Tyr Ile Pro Leu
            20

<210>  30
<211>  21
<212>  PRT
<213>  Artificial

<220>
<223>  A HPV L2-derived peptide to which three alanines are added at its
       N-terminal.

<400>  30

Ala Ala Ala Leu Gly Ile Gly Thr Gly Ser Gly Thr Gly Gly Arg Thr
1               5                   10                  15

Gly Tyr Ile Pro Leu
            20

<210>  31
<211>  21
<212>  PRT
<213>  Artificial

<220>
<223>  A HPV L2-derived peptide to which five alanines are added at its
       N-terminal.

<400>  31

Ala Ala Ala Ala Ala Ile Gly Thr Gly Ser Gly Thr Gly Gly Arg Thr
1               5                   10                  15

Gly Tyr Ile Pro Leu
            20

<210>  32
<211>  21
<212>  PRT
<213>  Artificial

<220>

<223>  A HPV L2-derived peptide to which nine alanines are added at its
      N-terminal.

<400>  32

Ala Ala Ala Ala Ala Ala Ala Ala Ala Ser Gly Thr Gly Gly Arg Thr
1               5                   10                  15

Gly Tyr Ile Pro Leu
            20